Europäisches Patentamt

European Patent Office

Office européen des brevets

(11) Veröffentlichungsnummer: **0 490 103 A1**

# (12) EUROPÄISCHE PATENTANMELDUNG

(21) Anmeldenummer: **91119301.9**

(22) Anmeldetag: **13.11.91**

(51) Int. Cl.5: **A61K 7/48**

(30) Priorität: **08.12.90 DE 4039244**

(43) Veröffentlichungstag der Anmeldung:
**17.06.92 Patentblatt 92/25**

(84) Benannte Vertragsstaaten:
**BE CH FR IT LI NL**

(71) Anmelder: **Glienke, Peter O.**
**120, Rue Principale**
**F-67470 Wintzenbach-Seltz(FR)**
Anmelder: **Gleinke, Isolde M.**
**120, Rue Principale**
**F-67470 Wintzenbach-Seltz(FR)**

(72) Erfinder: **Glienke, Peter O.**
**120, Rue Principale**
**F-67470 Wintzenbach-Seltz(FR)**
Erfinder: **Gleinke, Isolde M.**
**120, Rue Principale**
**F-67470 Wintzenbach-Seltz(FR)**

(74) Vertreter: **Trappenberg, Hans**
**Wendtstrasse 1**
**W-7500 Karlsruhe 21(DE)**

(54) **Peeling-Zubereitung.**

(57) Peeling-Zubereitungen sollten nicht nur Reinigen beziehungsweise Abtragen und Massieren, sondern auch von Laien ohne Verletzungsgefahr angewendet werden können wie auch bakterizid ausgestattet sein.

Diese Forderungen werden erfüllt durch eine kosmetische Peeling-Zubereitung, bei der der abrasive und gleichzeitig massierende Zusatz gebrochenes, geschäumtes Perlit ist, dessen Bruchstücke auf maximal 500 my klassiert sind, denen gegebenenfalls noch massierende Wachskügelchen und Bakterizide beigegeben sind.

EP 0 490 103 A1

Die Erfindung betrifft eine kosmetische Peeling-Zubereitung als Öl-in-Wasser-Emulsion oder Wasser-in-Öl-Emulsion in Form von Pasten, Gele, Lotions, Cremes etc. mit abrasiven und/oder massierenden Zusätzen wie Sägemehl, Sand, Bimsstein, gemahlenes Kaolin, gemahlener Kalkstein oder geschäumtem Perlit, also Perlitkügelchen aus auf Korngröße zerkleinertem und kurzfristig einer Temperatur von 1.300 K ausgesetzten Perlit-Gestein sowie mit bakteriziden und hautpflegenden Zusätzen.

In der Kosmetik wird seit längerer Zeit ein Verfahren zur intensiven Hautreinigung, das als "peeling"-Verfahren bekannt ist, angewendet. Bei diesem Verfahren werden insbesondere die oberflächlichen hornähnlichen Substanzen der Haut, wie auch die abgestorbenen Epidermiszellen entfernt, insbesondere bei oberflächlichen Verletzungen der Haut oder bei unschöner Ausbildung oder Vernarbung auch Teile der oberflächlichen Epidermis. Dieses intensive Reinigen beziehungsweise Abtragen oder Ablösen oberflächlicher Hautschichten darf selbstverständlich tatsächlich nur oberflächlich erfolgen, also nur innerhalb der obersten Hornschicht (stratum corneum); die daruntergelegenen Schichten, insbesondere die Keimschicht, darf hierbei keinesfalls verletzt werden. Zu beachten ist, daß bereits nach dem Abtragen der oberflächlichen Hornschicht eine deutlich erhöhte Entzündungsgefahr der Haut gegeben ist, da deren oberste Schutzschicht entfernt wurde und somit dem Eindringen von Krankheitskeimen weniger Widerstand entgegengesetzt wird. Es ist daher streng auf eine antiseptische Behandlungsweise zu achten beziehungsweise darauf, daß die nunmehr mehr oder weniger ungeschützten Hautpartien schnellstmöglich mit einer keimtötenden Substanz abgedeckt werden.

Üblicherweise wird dies dadurch erreicht, daß den bekannten Reinigungsmitteln neben Haut-Nährstoffen auch Fett und Fettemulsionen beigegeben werden, die diese abrasiv behandelten Hautpartien abdecken. Das Beifügen von keimtötenden Mitteln wird nicht immer vorgenommen, da diese Mittel die Haut reizen und als unangenehm empfunden werden können.

Allgemein gibt es für dieses "peeling"-Verfahren drei Gruppen von Reinigungsmitteln, solche die auf chemischer Basis die hornähnlichen Substanzen entfernen, weitere die hierfür Enzyme einsetzen und schließlich auch noch Reinigungsmittel, die rein mechanisch dieses Abtragen der hornähnlichen Substanzen beziehungsweise der abgestorbenen Epidermiszellen bewirken. Selbstverständlich sind auch Reinigungsmittel auf dem Markt, die kombiniert wirken, insbesondere solche, die den chemischen mit dem abrasiven Angriff verbinden.

Die kosmetische Behandlung der Haut kann bei diesem Reinigungsvorgang noch dadurch verbessert werden, daß die Haut gleichzeitig massiert und als Folge davon auch gut durchblutet wird. Diese Massage wird bei den bekannten Reinigungsmitteln durch das den Reinigungsmitteln beigefügte körnige Material unterstützt. Da dieses körnige Material, beispielsweise Bimsstein, jedoch unnachgiebig ist, wird diese Massagewirkung meistens als unangenehm empfunden.

Die Erfindung stellt sich die Aufgabe, eine kosmetische Peeling-Zubereitung anzugeben, die zwar auf abrasivem Wege sowohl die oberflächlichen hornähnlichen Substanzen der Haut und abgestorbene Epidermiszellen entfernt, wie auch erlaubt, oberflächlich die Haut-Oberschicht abzutragen, die gleichzeitig die behandelten Hautflächen beim Auftragen sanft massiert, die jedoch verhindert, daß dieses oberflächliche Abtragen zu tief bis zur Keimschicht der Haut gehen kann und die auch bereits beim Vorgang des Abtragens keimtötend wirkt.

Erreicht wird dies nach der Erfindung dadurch, daß das geschäumte Perlit Perlit-Bruchstücke, also gebrochene Perlitkügelchen sind, die auf maximal 500 μ klassiert sind.

Perlit (Perlitstein) ist ein natürliches wasserhaltiges Gesteinsglas, das aus etwa rundlichen Kügelchen besteht, die aus zwiebelähnlich sich umhüllenden Schalen zusammengesetzt sind. In diesen Kügelchen ist Wasser etwa mit 3 bis 6 Vol% in feinstverteilter Form fest eingeschlossen. Wird nun dieses Ausgangsmaterial, das Perlit-Gestein, auf Korngröße zerkleinert und kurzfristig einer Temperatur von etwa 1.300 K ausgesetzt und damit zum Schmelzen gebracht, so verwandelt sich das eingeschlossene Wasser in Dampf und bläht die Glasschmelze auf ein Vielfaches ihres ursprünglichen Volumens auf. Die so entstehenden expandierten Perlitkügelchen sind stark zerklüftet und weisen, durch ihre Entstehung bedingt, zahlreiche offene Kapillargänge auf. Die Festigkeit dieses Materials wird bei einer Schüttdichte zwischen 40 und 100 kg/m$^3$ mit etwa 0,005 bis 0,01 MN/m$^2$ angegeben, ist also so hoch, daß die Kügelchen ohne zu zerfallen geschüttet und transportiert werden können, andererseits aber so gering, daß sie bei höheren Drücken, also bei Drücken, wie sie beim Reinigungsvorgang ausgeübt werden, zu abrasivem Pulver zerfallen. Die Korngröße dieses expandierten Perlits richtet sich nach der ursprünglichen Zerkleinerung des Perlit-Gesteins; üblich sind Körnungen zwischen feinem Perlit-Pulver und Durchmessern bis ca. 10 Millimeter.

Tatsächlich wurde derart geschäumter Perlit auch schon als Reinigungsmittel, beispielsweise anstatt Bimsstein, eingesetzt. So schlägt eine tschechische Patentschrift 225 459 (CA 105 (2)-:8366f) unter anderem auch geschäumtes Perlit oder Sägemehl, Sand, gemahlenes Kaolin oder gemahlenen Kalkstein als abrasives oder massierendes Material vor. Die erhoffte gute, reinigende, abrasive und massierende Wirkung eines Perlitzusatzes hat sich allerdings nicht eingestellt. Dies dürfte, wie eingehende Untersuchungen gezeigt haben, darauf zurückzuführen sein, daß die aufgeschäumten kugelförmigen Perlitteilchen, möglicherweise auch weil sie mit Zubereitungs-Substanzen gefüllt waren, so fest waren, daß sie sich beim Auftragen des Reinigungsmittels nicht zerdrücken ließen, also keine scharfen abrasiven Kanten entstanden und auch die in ihnen enthaltenen Substanzen nicht freigelegt wurden. Dem versuchte man dadurch abzuhelfen, daß immer mehr dieses Perlitmaterials eingesetzt wurde, was letztendlich aber dazu führte, daß nicht akzeptable Verletzungen der Haut stattfanden. Erst die erfindungsgemäße Erkenntnis, daß nicht beliebig große kugelförmige Perlitteilchen in dem Reinigungsmittel eingesetzt werden dürfen, sondern Perlitbruchstücke bis zu einer maximalen Größe von 500 my, brachte den Erfolg. Nach wie vor sind diese Perlitbruchstücke zerklüftet und mit Kapillargängen versehen, so daß diese Perlitbruchstücke ebenso wie die zuvor eingesetzten Perlitkügelchen in der Lage sind, die Zubereitungs-Substanzen zu binden. Sie sind jedoch wesentlich druckempfindlicher, zerfallen also bereits bei leicht massierendem Auftragen zu Perlitpulver, wobei nicht nur die Zubereitungs-Substanzen freigegeben werden, sondern sich auch immer wieder, allerdings sehr feine, abrasive Kanten ergeben, die der gestellten Aufgabe genügen, also die gewünschte oberfläche Abtragung bewirken, ohne jedoch eine Verletzung herbeizuführen.

Bei dieser Gelegenheit ist auch noch auf einen weiteren Effekt hinzuweisen. Dadurch, daß die Oberfläche dieser Perlitbruchstücke stark zerklüftet ist, ist die gesamte freie Fläche gegenüber bekannten Sägemehlkörnungen oder mineralischen Körnungen etc. wesentlich höher, womit also an diesen freien Flächen deutlich mehr Zubereitungs-Substanz haften kann als an den bekannten Materialien. Diese Tatsache bringt mehrere Vorteile: Da mehr Zubereitungs-Substanz pro Volumeneinheit in der Zubereitung enthalten ist, ist die Reinigungswirkung deutlich verbessert beziehungsweise es wird weniger Reinigungsmittel benötigt. Damit ist aber auch der Anfall von abzuführenden Feststoffen deutlich geringer, und da diese Feststoffe, im wesentlichen das zerdrückte expandierte Perlit, pulverförmig sind, sind sie auch leicht wegzuschwemmen.

Ein außerordentlicher Vorteil kann darin gesehen werden, daß die Zubereitung, unter Anwendung entsprechender Verfahren, schüttfähig, damit also äußerst einfach dosierbar ist. Trotzdem wird die Zubereitung nach der Erfindung sofort bei der Anwendung, also beim Zerdrücken der Perlitbruchstücke, durch die austretenden Zubereitungs-Substanzen pastös, erhält also die zur Reinigung notwendige beziehungsweise gewünschte Konsistenz.

Zu dieser reinigenden und abrasiven wie auch massierenden Wirkung der Perlitbruchstücke gesellen sich nun noch die Wirkungen der Wachskügelchen beziehungsweise auch gegebenenfalls des beigefügten Bakterizids. Diese Wachskügelchen massieren wie gewünscht sanft die Haut und bringen gleichzeitig nicht nur ein verbessertes Gleitvermögen der Zubereitung auf der Haut, sondern decken auch die von den hornähnlichen Substanzen und der abgestorbenen Epidermis befreite Haut so ab, daß zumindest das Eindringen von Erregern gehemmt ist. Das Auftragen der erfindungsgemäßen Zubereitung wird also einfacher und für die behandelte Person angenehmer und führt auch gleichzeitig dazu, daß Entzündungen der behandelten Hauptpartien vermieden werden. Der guten kosmetischen Wirkung wegen wird vorgeschlagen, daß diese Wachskügelchen aus Jojobawachs sind. Sie können selbstverständlich jedoch auch aus sonstigen Wachsen, insbesondere Kunststoffwachsen, also beispielsweise Olefin-Polymerisaten, sein. Wichtig ist, daß diese Wachskügelchen den Durchmesser von 500 my nicht überschreiten und deren Erweichungstemperatur über 60 °C, also deutlich über der Temperatur der behandelten Haut, liegt.

Hinzu kommt noch die Wirkung des Bakterizids, das bereits beim Reinigungs- beziehungsweise Abtragungsvorgang auf die Hautoberfläche einwirkt und, bei richtiger Wahl des Bakterizids, sogar auch geringfügig in die oberste Hautschicht eindringt. Nach der Erfindung wird vorgeschlagen, daß als Bakterizid ein Naturstoff, nämlich Propolis, verwendet wird, vorzugsweise in Form eines bioverfügbaren Propolis-Extraktes.

Propolis ist ein Harz, das aus einer großen Menge von verschiedenartigen Stoffen besteht, die von den Bienen in den Bienenstock eingetragen werden. Mit Propolis verkitten die Bienen ihre Behausung, verstärken ihre Waben und glätten rauhe Teile des Stockes. Außerdem werden die Waben, wie auch sämtliche Behausungsteile, mit einem dünnen Propolisfilm überzogen, der nach neueren Forschungen dafür verantwortlich ist, daß bei Bienen trotz der dichten Bevölkerung in einem Bienenstock keine Seuchen auftreten.

Von dieser antibakteriellen Eigenschaft ausgehend wurde die Propolis auch schon im Altertum als insbesondere äußerlich anzuwendendes Heilmittel gebraucht. Diesem Gebrauch kommt die wachs- bis harzartige Konsistenz der Propolis entgegen. Für die Anwendung nach der Erfindung sollte jedoch die Propolis in Lösung sein, um sie biologisch verfügbar zu machen. Dies wird beispielsweise erreicht durch ein biologisch verfügbares Propolis-Extrakt nach der DE-OS 32 10 272.

Dieses aufbereitete Propolis-Extrakt verteilt sich in einem dünnen Film auf der gereinigten Hautoberfläche und schützt somit diese Hautoberfläche zuverlässig gegen den Angriff von Krankheitskeimen. Zusätzlich wandern Teile dieses Propolis-Extraktes in die Hautoberfläche ein und entfalten dort nicht nur ihre antibakterielle Wirkung, sondern vermitteln der Haut, wie praktische Versuche gezeigt haben, auch weitere Aufbaustoffe.

Zusammenfassend kann gesagt werden, daß durch die Verwendung von expandierten Perlitbruchstücken für die Zubereitung nach der Erfindung, wie auch durch die Beimengung der Wachskügelchen und eines Bakterizids, insbesondere eines Propolis-Extraktes, eine deutliche Verbesserung der Massage- und Reinigungswirkung erzielt wird, die zudem noch durch die Wahl der jeweiligen Korngröße so begrenzt ist, daß Verletzungen nicht stattfinden können und daß bei Anwendung des Reinigungsmittels, insbesondere bei tiefem Abtragen, Entzündungen nicht zu befürchten sind.

Die Zubereitungs-Substanzen wie auch das Bakterizid werden zweckmäßigerweise in flüssiger Form mit dem expandierten Perlit vermengt. Sollten diese Substanzen nicht in flüssiger Form vorliegen, so empfiehlt es sich, sie beispielsweise durch Wärmeeinwirkung zu verflüssigen. Bei manchen Substanzen ist es auch noch zweckmäßig, um eine Verflüchtigung zu vermeiden, oder auch um eine Verfestigung und damit eine verstärkte Massagewirkung der expandierten Perlitbruchstücke zu erzielen, die Perlitbruchstücke mit einer Wachs- oder Wasserglasumhüllung zu versehen, wobei die Wachsumhüllung einen Erweichungspunkt aufweisen soll, der etwa mit der Hauttemperatur übereinstimmt, also unter 40 °C liegt.

Schließlich kann die Zubereitung auch noch, zweckmäßigerweise nach dem Transport, mit Polyäthylenglykol vermengt werden, um es in eine angenehme Darreichungsform zu bringen. Selbstverständlich können vor der Anwendung auch noch weitere Substanzen beigemengt werden, insbesondere Duftstoffe oder sonstige für die Reinigung oder die Darreichungsform zweckmäßige flüssige oder auch pulverförmige Materialien.

Die erfindungsgemäße Zubereitung soll an einem Rezeptbeispiel erläutert werden:

| | |
|---|---|
| 78 GW% | Aloe Vera Gel |
| 2 GW% | Betainmonohydrat |
| 5 GW% | Jojoba-Wachskügelchen in der Korngröße 40/60 my |
| 2,92 GW% | kosmetischer Wirkstoffkomplex aus Biohyaluronsäure (Natriumsalz) und Liposomen |
| 0,8 GW% | Konservierungsmittel |
| 2 GW% | Gelbildner (Polysacharit-Xanthan-Gum) |

| | |
|---|---|
| 2 GW% | bioverfügbares Propolis-Extrakt werden mit |
| 8 GW% | Perlitbruchstücke der definierten Größe und Struktur vermengt. |

Die Vermengung erfolgt im Tauchverfahren unter Vakuum, so daß die flüssigen Substanzen mit dem Propolis-Extrakt durch Kapillarwirkung und van-der-Waals-Kräfte vollkommen in die Kapillargänge der expandierten Perlitbruchstücke eindringt, wie auch an deren zerklüfteten Oberfläche haftet. Nach dem Beifügen von Duftstoffen wird das gefüllte Trägermaterial abgezogen, kurz auf einem Bandtrockner getrocknet und sodann dosiert. Damit liegt die Zubereitung in verbrauchsfertiger Form vor, da beim Gebrauch durch Zerdrücken der Perlitbruchstücke die Substanzen, wie auch das Propolis-Extrakt, freigegeben werden und zusammen mit den abrasiven Perlitbruchstücken und den Wachskügelchen eine äußerst gute Massage und Reinigungswirkung entfaltet.

**Patentansprüche**

1. Kosmetische, halbflüssige Peeling-Zubereitung als Öl-in-Wasser-Emulsion oder Wasser-in-Öl-Emulsion in Form von Pasten, Gele, Lotions, Cremes etc. mit abrasiven und/oder massierenden Zusätzen wie Sägemehl, Sand, Bimsstein, gemahlenes Kaolin, gemahlener Kalkstein oder geschäumtem Perlit, also Perlitkügelchen aus auf Korngröße zerkleinertem und kurzfristig einer Temperatur von etwa 1.300 K ausgesetzten Perlit-Gestein sowie mit bakteriziden und hautpflegenden Zusätzen,
dadurch gekennzeichnet,
daß das geschäumte Perlit Perlit-Bruchstücke, also gebrochene Perlitkügelchen sind, die auf maximal 500 μ klassiert sind.

2. Zubereitung nach Anspruch 1,
dadurch gekennzeichnet,
daß dem Gemenge Wachskügelchen mit einem Durchmesser bis maximal 500 my und einem über 40 °C liegenden Erweichungspunkt zugefügt sind.

3. Zubereitung nach Anspruch 2,
dadurch gekennzeichnet,
daß die Wachskügelchen aus Jojobawachs sind mit einem bei 50 my liegenden Durchmesser.

4. Zubereitung nach Anspruch 2,
dadurch gekennzeichnet,
daß die Wachskügelchen aus niedermolekularen Olefin-Polymerisaten (Alkene) sind.

5. Zubereitung nach Anspruch 1,

dadurch gekennzeichnet,
daß das Bakterizid Propolis (Kittharz der Bienen) ist.

6. Zubereitung nach Anspruch 5,
dadurch gekennzeichnet,
daß die Propolis als bioverfügbarer Propolisextrakt dem Gemenge beigegeben ist.

7. Reinigungsmittel nach Anspruch 1,
dadurch gekennzeichnet,
daß die mit Zubereitungssubstanzen gefüllten Perlitbruchstücke mit einem Mantel aus unter 40 ºC schmelzenden Wachs versehen sind (coated particels).

8. Reinigungsmittel nach Anspruch 1,
dadurch gekennzeichnet,
daß die mit Zubereitungssubstanzen gefüllten Perlitbruchstücke mit einem Mantel aus Wasserglas versehen sind (coated particels).

| | Europäisches Patentamt | EUROPÄISCHER RECHERCHENBERICHT | Nummer der Anmeldung |
|---|---|---|---|

EP 91 11 9301

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | DE-C-1 289 600 (EKOPERL GMBH) <br> * das ganze Dokument * <br> --- | 1 | A61K7/48 |
| A | GB-A-2 178 441 (KANFER) <br> * Ansprüche * <br> --- | 1 | |
| A | PATENT ABSTRACTS OF JAPAN <br> vol. 12, no. 467 (C-550)(3314) <br> & JP-A-63 188 611 ( NOEBIA K.K. ) <br> * Zusammenfassung * <br> --- | 1 | |
| A,D | DE-A-3 210 272 (FA. PETER O. GLIENKE + PARTNER INDUSTRIEBERATUNG GMBH) <br> * das ganze Dokument * <br> ----- | 1,5-6 | |
| | | | **RECHERCHIERTE SACHGEBIETE (Int. Cl.5)** <br><br> A61K |

Der vorliegende Recherchenbericht wurde für alle Patentansprüche erstellt

| Recherchenort | Abschlußdatum der Recherche | Prüfer |
|---|---|---|
| DEN HAAG | 30 JANUAR 1992 | FISCHER J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
...................................................
& : Mitglied der gleichen Patentfamilie, übereinstimmendes Dokument

EPO FORM 1503 03.82 (P0403)